# EUROPÄISCHE PATENTSCHRIFT

(11) **EP 2 453 795 B1**
(45) Veröffentlichungstag und Bekanntmachung des Hinweises auf die Patenterteilung: **20.09.2017**
(21) Anmeldenummer: 10720893.6
(22) Anmeldetag: 30.04.2010
(51) Int. Cl.: A61B 5/151, F16D 7/02

(54) **ANALYTISCHES TESTGERÄT MIT RUTSCHKUPPLUNG**
Analytical tester with slip clutch
Appareil de test analytique doté d'un accouplement à patinage

(30) Priorität: 14.07.2009 EP 09165425
(43) Veröffentlichungstag der Anmeldung: 23.05.2012
(73) Patentinhaber: Roche Diabetes Care GmbH, 68305 Mannheim (DE); F. Hoffmann-La Roche AG, 4070 Basel (CH)
(72) Erfinder: KONYA, Ahmet, 67065 Ludwigshafen (DE)
(74) Vertreter: Herzog, Fiesser & Partner Patentanwälte PartG mbB
(86) Internationale Anmeldenummer: PCT/EP2010/055906
(87) Internationale Veröffentlichungsnummer: WO 2011/006690

(56) Entgegenhaltungen:
- EP-A2- 0 905 400
- WO-A-2008/138443
- WO-A-2009/037341
- DE-A1- 10 315 808
- DE-U1-202005 000 215
- FR-A- 2 353 753
- GB-A- 2 321 504
- GB-A- 2 404 703
- US-A- 3 092 983

## Beschreibung

### Gebiet der Erfindung

Die Erfindung betrifft ein analytisches Testgerät mit mindestens einer Rutschkupplung sowie ein Bandmagazin zum Einsatz in einem derartigen analytischen Testgerät. Derartige analytische Testgeräte und Bandmagazine werden insbesondere im Bereich der medizinischen Diagnostik eingesetzt, insbesondere im Rahmen eines Nachweises mindestens eines Analyten in einer Körperflüssigkeit. Insbesondere kann es sich bei diesem Analyten um Glukose handeln, beispielsweise Blutglukose und/oder Glukose in interstitieller Flüssigkeit. Auch andere Einsatzgebiete sind jedoch grundsätzlich möglich.

### Stand der Technik

In der medizinischen Diagnostik, jedoch auch in anderen Bereichen, tritt die Aufgabenstellung auf, dass schnell und zuverlässig und teilweise mehrfach am Tage bestimmte Analyte nachgewiesen werden müssen. Beispielsweise müssen Diabetiker in vielen Fällen bis zu 7-mal täglich ihren Blutglukosegehalt überprüfen. Um durch diese häufigen Kontrollen den Tagesablauf der Benutzer nicht mehr als nötig einzuschränken, wurden Handgeräte entwickelt, welche die Kontrolle der Blutglukosegehalte auch in der Freizeit oder am Arbeitsplatz ermöglichen. Dabei wird mittels einer Lanzette ein Einstich in eine Haut des Benutzers erzeugt, beispielsweise einer Fingerbeere oder einem Ohrläppchen. Auf diese Weise wird eine Blutprobe oder eine Probe interstitieller Flüssigkeit generiert. Diese wird dann mittels eines Testgeräts und eines Testelement, beispielsweise eines elektrochemischen und/oder optischen Testelements, auf ihren Blutglukosegehalt und/oder ihren Gehalt an einer anderen Art von Analyt untersucht.

Neben Einzelstreifen-Testgeräten sind mittlerweile auch Bandgeräte bekannt, bei welchem Testelemente in Form von Testbändern bereitgestellt werden. Diese Testbänder enthalten eine Mehrzahl von Testfeldern mit einer entsprechenden Testchemie, deren Eigenschaften sich bei Kontakt mit dem nachweisenden Analyten ändern. Neben analytischen Testgeräten mit Analysebändern in Form von Testbändern sind mittlerweile auch analytische Testgeräte mit Analysebändern bekannt, welche mehrere Lanzetten enthalten. Dabei werden nacheinander Lanzetten auf einem Trägerband in einer Applikationsposition des Testgeräts bereitgestellt und können für die Probengenerierung verwendet werden. Beispielsweise kann das Analyseband von einem Gutwickel bereitgestellt werden, wobei benutzte analytische Hilfsmittel, beispielsweise Testfelder und/oder Lanzettenelemente, nach Benutzung auf einen Schlechtwickel aufgespult werden.

WO 2008/138443 A1 zeigt ein Stechsystem mit einem Trägerband, welches mehrere Lanzetten trägt. Weiterhin ist eine Fördereinrichtung vorgesehen, um die Lanzetten durch Bewegen des Trägerbandes in einer Förderrichtung nacheinander in eine Applikationsposition zu bringen. Zudem ist eine Betätigungsvorrichtung zum Antreiben der Fördereinrichtung vorgesehen. Weiterhin umfasst das Stechsystem eine lösbare Sperre, die einen Weitertransport des Trägerbandes blockiert, sobald eine Lanzette die Gebrauchsposition erreicht hat.

Aus WO 2009/037341A1 ist ein Probengewinnungssystem zum Gewinnen einer flüssigen Probe bekannt, welches mindestens ein analytisches Hilfsmittel aufweist. Das Probengewinnungssystem weist ein Kopplungselement zum Ankoppeln an das analytische Hilfsmittel sowie mindestens einer Antriebseinheit zum Antreiben einer Bewegung des Kopplungselements aus einer Ruheposition in eine ausgelenkte Position auf. Die Antriebseinheit umfasst einen Energiewandler, welcher ausgestaltet ist, um eine Drehbewegung mit unterschiedlichen Drehrichtungen zu erzeugen. Die Antriebseinheit weist weiterhin eine Kopplungsvorrichtung mit mindestens einem drehrichtungssensitiven Element auf, welche eingerichtet ist, um in einer ersten Drehrichtung den Energiewandler an eine erste Systemfunktion und an einer zweiten, von der ersten Drehrichtung verschiedenen Drehrichtung an eine zweite, von der ersten Systemfunktion verschiedene Systemfunktion anzukoppeln.

Sowohl in WO 2008/138443 A1 als auch in WO 2009/037341A1 werden aus verschiedenen Gründen so genannte Rutschkupplungen in verschiedenen Ausführungsformen offenbart, insbesondere in einem Antrieb für die Analysebänder. Diese Rutschkupplungen können beispielsweise Spiralfederelemente umfassen. Die in diesen Druckschriften offenbarten Rutschkupplungen basieren dabei allesamt darauf, dass ein oder mehrere federnde, elastische oder federgelagerte Arme oder andere Arten elastischer Mitnehmerelemente wie beispielsweise Spiralfedern punktförmig auf einer Reibfläche eines Gegenelements gleiten und mit Vorsprüngen dieses Gegenelements zusammenwirken. Die Mitnehmerelemente bleiben an diesen Vorsprüngen hängen und nehmen diese mit, solange ein maximales Drehmoment nicht überschritten wird. Dieses maximale Drehmoment ist durch die Elastizität und Formgestaltung der Mitnehmerelemente und Vorsprünge gegeben, denn mit zunehmendem Drehmoment werden die Mitnehmerelemente von der Reibfläche des Gegenelements und/oder den Vorsprüngen weggedrückt und gleiten ab dem maximalen Drehmoment über diese Vorsprünge hinweg. Dies bedeutet jedoch, dass das maximale Drehmoment oder Grenz-Drehmoment fest durch die Geometrie der Vorsprünge und der Mitnehmerelemente sowie die Materialeigenschaften und Biegemomente der Mitnehmerelemente vorgegeben ist und insbesondere nicht eingestellt werden kann. Weiterhin tritt bei dem Gleiten der Mitnehmerelemente über die Vorsprünge eine erhebliche Geräuschentwicklung auf. Derartige Feder-basierte Rutschkupplungen werden häufig auch als Freiläufe bezeichnet oder sind als Freiläufe ausgestaltet.

Beispielsweise benötigt die in WO 2009/037341A1 dargestellte Stechsystem-Konfiguration mit der Fördereinrichtung in der Regel aus drei Gründen mindestens eine Rutschkupplung. So kann eine Rutschkupplung vorgesehen sein, um Toleranzen auszugleichen, die sich durch die Fertigung der Analysebänder ergeben. Weiterhin ist die Rutschkupplung vorgesehen, um die Variation des Schlechtwickeldurchmessers auszugleichen, der bei einem Weitertaktvorgang zunimmt. Drittens ist die Rutschkupplung eingerichtet, um eine mechanische Verletzung des Bandes zu verhindern, da, wenn die Lanzette die Gebrauchsposition erreicht hat, ein unbeabsichtigter Weitertransport zu einem Ablösen der Lanzette beziehungsweise einer Verformung des Analysebandes führen könnte. Die Rutschkupplung sorgt dafür, dass, bevor eine derartige mechanische Beschädigung des Bands auftritt, ein Lösen des Antriebs durch ein Durchrutschen erfolgt, so dass eine mechanische Überbeanspruchung vermieden wird. Beispielsweise können hierfür Spiralfedern verwendet werden.

Insbesondere bei der Beförderung von Lanzettenbändern, jedoch auch bei anderen Arten von Analysebändern, tritt beispielsweise aufgrund der Aufwicklung des Bandes eine Veränderung der Rollendicke sowohl eines Gutwickels als auch eines Schlechtwickels auf, so dass bei gleicher Kraftwirkung pro Weitertaktung unterschiedliche Fahrwege des Bandes zu überwinden sind. Ist der Fahrweg der Rolle sehr kurz, so muss die überschüssige Kraft abgeführt werden. Dies wird beispielsweise durch die Rutschkupplung erreicht. Dabei können beispielsweise Metallfedern vorgesehen sein, welche ständig unter einem bestimmten Druck stehen, wodurch ein ständiger Zug auf das Band ausgeübt wird.

Die aus dem Stand der Technik bekannten Systeme weisen jedoch insbesondere für den Einsatz von Lanzettenbändern verschiedene Nachteile oder technische Herausforderungen auf. So ist beispielsweise in WO 2008/138443 A1, wie oben dargestellt, eine lösbare Sperre, beispielsweise in Form eines Greifers, vorgesehen, welcher sowohl dazu dient, das Band anzuhalten, wenn die Lanzette an ihm vorbei transportiert wird, welche jedoch auch zum Einstichvorgang benutzt wird. Bei der in dem Stand der Technik bekannten Rutschkupplungen wirkt jedoch auf das Analyseband eine permanente Kraft, beispielsweise durch die vorgespannten Spiralfedern. Diese Spiralfedern, beispielsweise Metallfedern, stehen ständig unter einem bestimmten Druck, wodurch ein ständiger Zug auf das Band ausgeübt wird. Hierdurch muss auch der Anwender beispielsweise in dem in WO 2008/138443 A1 beschriebenen System mehr Kraft aufbringen, um das System zu bedienen, was nachteilig sein kann. Ein weiterer Nachteil ist die akustische Komponente, da ein minimaler Totgang realisiert werden muss, wodurch eine unangenehme Geräuschkulisse entstehen kann. Dies basiert auf der Funktionsweise einer Federrutschkupplung, welche sich zusammenzieht und über Hindernisse durchrutscht. Jedes Überwältigen eines Hindernisses lässt ein Geräusch entstehen. Um möglichst wenig Totgang zu erreichen, werden jedoch mehrere dieser Hindernisse, auf 360 C° verteilt, benötigt. Ein weiterer Nachteil besteht darin, dass es bei bekannten Spiralfedersystemen schwierig ist, Federn bereitzustellen, deren Bauraum in einem Wickelkern aufgenommen werden kann, wodurch unter Umständen die Gesamtgröße des Systems auf Grund dieser Komponente wächst.

Zusammenfassend weisen bekannte Feder-Rutschkupplungen für Testgeräte also den Nachteil eines Totgangs auf, welcher ein exaktes Greifen der Rutschkupplung in vielen Fällen nicht ermöglicht. Weiterhin tritt durch das punktuelle Zusammenwirken der Mitnehmerelemente und Vorsprünge eine erhebliche Geräuschentwicklung auf, die insbesondere beim Anwender für Verunsicherung sorgen kann und den Verwendungskomfort erheblich einschränken kann. Weiterhin sind die maximalen Drehmomente der Rutschkupplungen in der Regel durch Geometrien und Werkstoffeigenschaften der Mitnehmerelemente und/oder Vorsprünge fest vorgegeben und nicht einstellbar.

Aus dem schweren Maschinenbau sind grundsätzlich Rutschkupplungen bekannt. So beschreibt beispielsweise die DE 10315808 A1 eine Rutschkupplung mit einer hülsenförmigen Nabe zur Aufnahme eines Antriebselements und einem endständigen Klemmteil mit einem im Bereich einer radialen Mittelebene sich erstreckenden Schlitz und mit einer den Schlitz überbrückenden, in Umfangsrichtung des Klemmteils wirkenden Klemmschraube. Ein Antriebselement ist zwischen zwei Reibbelägen angeordnet, die ihrerseits axial zwischen einer Druckscheibe und dem Klemmteil angeordnet sind und mittels Federkraft über eine Nachstellmutter einstellbar beaufschlagt sind. Das Klemmteil ist einteilig mit der hülsenförmigen Nabe ausgeführt.

EP 0905400 beschreibt eine Rutschkupplung mit einem Gummiring, welcher in axialer Richtung zwischen einer äußeren Antriebsscheibe und einer inneren Nabe angeordnet ist.

GB 2321504 A beschreibt eine Rutschkupplung mit einem ersten Ring und zwei inneren Ringen, welche jeweils frusto-konische Kupplungsoberflächen aufweisen, die ineinander eingreifen. Der Betrag der erzeugten Reibungskraft, welche senkrecht zu den Kupplungsoberflächen ausgeübt wird, wird variiert mittels eines Einstellglieds.

Die letztgenannten, aus dem Stand der Technik bekannten Rutschkupplungen sind jedoch bislang lediglich aus dem Bereich des schweren Maschinenbaus bekannt. So wird die in DE 103 15 808 A1 beispielsweise zum Antrieb von Kettenrädern eingesetzt. Dementsprechend sind die bekannten Rutschkupplungen allgemein vergleichsweise schwer, aus vielen Teilen zusammengesetzt und weisen einen hohen Bauraum auf. Ein Bedarf, derartige Rutschkupplungen im Bereich der Medizintechnik, insbesondere im Bereich analytischer Testgeräte, einzusetzen, bestand bislang nicht.

### Aufgabe der Erfindung

Es ist daher eine Aufgabe der Erfindung, ein analytisches Testgerät und ein Bandmagazin zum Einsatz in einem derartigen analytischen Testgerät anzugeben, welche die Nachteile des Standes der Technik zumindest weitgehend vermeiden. Insbesondere soll dabei ein möglichst geräuschloser Transport eines Analysebands, ohne zusätzlich aufzubringende Kraft auf Grund eines Vorspannens gewährleistet werden, welcher einen möglichst geringen Bauraum aufweist und überdies kostengünstig ist.

### Offenbarung der Erfindung

Diese Aufgabe wird gelöst durch ein analytisches Testgerät, ein Bandmagazin und eine Verwendung mit den Merkmalen der unabhängigen Ansprüche. Vorteilhafte Weiterbildungen der Erfindungen, welche einzeln oder in Kombination realisierbar sind, sind in den abhängigen Ansprüchen dargestellt.

In einem ersten Aspekt der Erfindung wird ein analytisches Testgerät vorgeschlagen. Unter einem analytischen Testgerät ist allgemein eine Vorrichtung zu verstehen, welche im Rahmen einer Analyse eingesetzt werden kann, beispielsweise zum Nachweis eines oder mehrerer Analyte in einer Körperflüssigkeit. Das analytische Testgerät kann dementsprechend eine Probenamefunktion zum Generieren einer oder mehrer Proben und/oder eine Analysefunktion aufweisen. Im Folgenden wird im wesentlichen Bezug genommen auf analytische Testgeräte, welche ausschließlich eine Probenamefunktion in Form einer Lanzettenfunktion aufweisen. Alternativ oder zusätzlich können jedoch auch andere Arten von analytischen Testgeräten zum Einsatz kommen. Das analytische Testgerät kann insbesondere als Handgerät ausgestaltet sein.

Das analytische Testgerät basiert auf der Verwendung mindestens eines Analysebands, welches ganz oder teilweise in dem analytischen Testgerät aufgenommen ist. Beispielsweise kann dieses Analyseband Teil eines Bandmagazins sein, beispielsweise eines erfindungsgemäßen Bandmagazins gemäß einem weiteren, unten noch näher ausgeführten Aspekt der Erfindung.

Unter einem Analyseband ist dabei grundsätzlich ein beliebiges Band zu verstehen, welches sich im Rahmen einer Analyse, beispielsweise im Rahmen eines Nachweises eines oder mehrere Analyte in einer Körperflüssigkeit, eingesetzt werden kann. Dementsprechend kann das Analyseband beispielsweise eine Mehrzahl von analytischen Hilfsmitteln aufweisen, beispielsweise eine Mehrzahl von Testfeldern und/oder eine Mehrzahl von Lanzetten. Die Mehrzahl analytischer Hilfsmittel ist nacheinander in dem analytischen Testgerät einsetzbar. Die Testfelder können beispielsweise eine Testchemie aufweisen, welche bei Anwesenheit des mindestens einen nachzuweisenden Analyten mindestens eine messbare Eigenschaft ändert, beispielsweise eine physikalisch und/oder chemisch messbare Eigenschaft. Ohne Beschränkung weiterer möglicher Ausgestaltungen wird im folgenden insbesondere auf Lanzettenbänder Bezug genommen, also Analysebänder, welche ausschließlich Lanzetten als analytische Hilfsmittel umfassen. Auch andere Ausgestaltungen sind jedoch möglich, beispielsweise Ausgestaltungen, bei welchen das Analyseband sowohl Lanzetten als auch Testfelder aufweist.

Mittels der Transportvorrichtung kann das analytische Testgerät beispielsweise nacheinander die analytischen Hilfsmittel in mindestens eine Anwendungsposition bringen. Die Transportvorrichtung umfasst dabei mindestens eine Rutschkupplung. Unter einer Rutschkupplung ist dabei grundsätzlich eine beliebige Kupplung zu verstehen, welche eine Sicherheitsfunktion in Form einer selbsttätig drehmomentsschaltenden Schaltung aufweist. Eine Idee der Erfindung basiert darin, im Gegensatz zu dem oben zitierten Stand der Technik eine Rutschkupplung einzusetzen, welche auf Reibung basiert. Dementsprechend kann die Rutschkupplung erfindungsgemäß derart eingerichtet sein, dass diese bis zum Überwinden eines der Haftreibung entsprechenden Drehmoments einen Mitnahmeeffekt aufweist, wohingegen ab einem Drehmomentbereich, in welchem die Haftreibung überwunden ist, kein Mitnahmeeffekt mehr auftritt und lediglich noch eine Gleitreibung wirkt. Damit steht die vorgeschlagene Rutschkupplung im Unterschied zu den aus WO 2009/037341 A1 oder WO 2008/138443 A1 bekannten Rutschkupplungen, welche auf einem punktuellen Zusammenwirken von Mitnahmeelementen mit Vorsprüngen basieren.

Dementsprechend wird vorgeschlagen, dass die Rutschkupplung mindestens ein mit einem Antrieb koppelbares, insbesondere gekoppeltes, erstes Reibelement und mindestens ein mit dem Analyseband, insbesondere einem Wickel des Analysebands, koppelbares, insbesondere gekoppeltes, zweites Reibelement aufweist. Das erste Reibelement und das zweite Reibelement sind dabei derart reibschlüssig miteinander verbunden, dass das erste Reibelement in einem ersten Drehmomentbereich das zweite Reibelement mitnimmt, das heißt ein Drehmoment auf dieses überträgt, und in einem zweiten Drehmomentbereich, insbesondere einem größeren Drehmomentbereich als dem ersten Drehmomentbereich, über das zweite Reibelement hinweggleitet, das heißt kein oder lediglich ein geringes Drehmoment auf dieses überträgt. Der Reibschluss zwischen dem ersten Reibelement und dem zweiten Reibelement kann dabei grundsätzlich direkt oder indirekt erfolgen, wobei ein direkter Reibschluss einen unmittelbaren Kontakt zwischen entsprechenden Reibflächen des ersten beziehungsweise zweiten Reibelements beinhaltet, wohingegen ein indirekter Reibschluss einen mittelbaren Kontakt über mindestens ein Zwischenelement beinhaltet. Entsprechend kann auch das Hinweggleiten des ersten Reibelements über das zweite Reibelement in dem zweiten Drehmomentbereich direkt oder indirekt erfolgen, wobei ein indirektes Hinweggleiten ein Gleiten über mindestens ein Zwischenelement beinhaltet.

Allgemein kann eine scharfe Grenze zwischen dem ersten Drehmomentbereich und dem zweiten Drehmomentbereich vorgegeben sein oder auch ein kontinuierlicher Übergangsbereich. Die Grenze wird in der Regel, wie oben dargestellt, durch einen Übergang von der Haftreibung zur Gleitreibung vorgegeben, also beispielsweise durch einen Punkt, an welchem ein "Freibrechen" das ersten Reibelements von dem zweiten Reibelement oder umgekehrt erfolgt. Auch damit steht die vorgeschlagene Rutschkupplung im Unterschied zu den aus WO 2009/037341 A1 oder WO 2008/138443 A1 bekannten Rutschkupplungen, bei welchen kein Reibschluss sondern ein formschlüssiges Zusammenwirken zwischen einem Mitnahmeelement und mindestens einem Vorsprung erfolgt. Ein maximales Drehmoment oder Grenz-Drehmoment, also der Übergang zwischen dem ersten Drehmomentbereich und dem zweiten Drehmomentbereich, ist bei der vorgeschlagenen Rutschkupplung vorzugsweise durch den Übergang zwischen Haftreibung und Gleitreibung zwischen dem ersten Reibelement und dem zweiten Reibelement gegeben, insbesondere ausschließlich durch diesen Übergang zwischen Haftreibung und Gleitreibung, vorzugsweise ohne zusätzliche Einflussfaktoren wie beispielsweise Biegemomente oder Federkräfte.

Unter einem Reibschluss ist dabei ein Zusammenwirken zweier Elemente, im vorliegenden Fall des ersten Reibelements und des zweiten Reibelements, zu verstehen, bei welchem eine Mitnahme aufgrund von Reibungskräften erfolgt, vorzugsweise ausschließlich aufgrund von Reibungskräften. Insbesondere kann die Mitnahme derart erfolgen, dass das erste Reibelement und das zweite Reibelement auf zueinander zumindest lokal parallelen Reibflächen aufeinander aufliegen. Die Reibungskräfte können dann in der Ebene der parallelen Reibflächen wirken. Vorzugsweise sind keine mechanischen Mitnehmerelemente vorgesehen, auf welche unmittelbar Druck- und/oder Zugkräfte ausgeübt werden.

Der Reibschluss zwischen den Reibelementen erfolgt vorzugsweise kontinuierlich über den gesamten Winkelbereich der Rutschkupplung hinweg, also beispielsweise über 360°. Dies bedeutet, dass die Reibelemente über den gesamten Drehwinkelbereich der Rutschkupplung hinweg aufeinander aufliegen, im Gegensatz beispielsweise zu den aus WO 2009/037341 A1 oder WO 2008/138443 A1 bekannten Rutschkupplungen. Dabei können die Reibflächen vorzugsweise als über den gesamten Winkelbereich durchgängig gestaltete, geschlossene Reibflächen ausgestaltet sein. Alternativ können jedoch auch ein oder mehrere der aufeinander reibenden Reibflächen der Reibelemente diskontinuierlich ausgestaltet sein. Ein kontinuierlicher Reibschluss über den gesamten Drehwinkelbereich hinweg bewirkt, dass kein Totgang auftritt. Weiterhin lassen sich auf diese Weise Geräuschentwicklungen vermeiden. Insbesondere kann im Rahmen der vorliegenden Erfindung ein kontinuierlicher Aufbau des Drehmoments über den gesamten Winkelbereich, innerhalb dessen die Reibelemente zueinander verdreht werden können, realisierbar sein, im Gegensatz zu dem diskontinuierlichen Aufbau gemäß den in WO 2009/037341 A1 oder WO 2008/138443 A1 beschriebenen Rutschkupplungen, bei welchen lediglich an den Vorsprüngen eine Drehmomentüberwindung erfolgt.

Die Verwendung eines Reibschlusses anstelle eines formschlüssigen Zusammenwirkens von biegbareren Mitnehmerelementen mit einem oder mehreren Vorsprüngen und der damit verbundenen Ausübung von lokalen Zugkräften, wie beispielsweise in der WO 2009/037341 A1 oder WO 2008/138443 A1, bietet weiterhin den Vorteil, dass sich das maximale Drehmoment, ab welchem das Freibrechen erfolgt, sehr präzise einstellen lässt. So ist der Reibschluss in der Regel durch Anpresskräftte definiert, mit welchen die Reibelemente gegeneinander gepresst werden. Diese Anpresskräftte lassen sich jedoch sehr präzise vorgegeben oder sogar einstellen. Das erste Reibelement und das zweite Reibelement sind vorzugsweise im Bereich ihrer zusammenwirkenden Reibflächen über den gesamten Drehmomentbereich und/oder Winkelbereich unveränderlich in ihrer Form, im Gegensatz beispielsweise zur WO 2009/037341 A1 oder WO 2008/138443 A1.

Die Rutschkupplung ist zwischen einer angetriebenen Achse und einem die Achse umschließenden Element, insbesondere einem Wickel des Analysebands (beispielsweise einem Gutwickel und/oder einem Schlechtwickel) angeordnet. Die Reibelemente können sowohl aus dem gleichen Werkstoff ausgeführt werden als auch aus unterschiedlichen Werkstoffen. Bevorzugt ist lediglich das Vorliegen einer einstellbaren Grenze des Übergangs von der Haftreibung zur Gleitreibung. Das mindestens eine erste Reibelement und das mindestens eine zweite Reibelement können direkt aufeinander reiben und somit selbst die Reibflächen bereitstellen, zwischen denen der Reibschluss erfolgt. Alternativ oder zusätzlich sind, zwischen dem ersten Reibelement und dem zweiten Reibelement, wie unten noch näher ausgeführt wird, ein oder mehrere Zwischenelemente vorgesehen, wie beispielsweise der unten noch näher ausgeführte O-Ring. Der Reibschluss zwischen dem ersten Reibelement und dem zweiten Reibelement kann also über mindestens ein Zwischenelement, insbesondere mindestens einen O-Ring, erfolgen. Der Vorteil dieser Ausführungsvariante liegt in der Möglichkeit einer Bauraumeinspaarung, da beispielsweise der O-Ring die Vorspannkraft der Rutschkupplung aufbringen kann. Der O-Ring kann dann wie ein Federelement wirken, so dass kein separates Federelement erforderlich ist.

Wie oben dargestellt, ist die Rutschkupplung zwischen einer angetriebenen Achse und einem die Achse umschließenden Element insbesondere einem Wickel des Analysebands, angeordnet. Beispielsweise kann die Rutschkupplung teilweise oder vollständig von dem Wickel umgeben sein. Beispielweise kann diese angetriebene Achse als Teil der Transportvorrichtung und/oder als Teil eines Bandmagazins ausgestaltet sein. Der Antrieb kann beispielsweise über mindestens einen Antriebsriemen und/oder über mindestens ein Zahnrad erfolgen. Bei dem Wickel des Analysebandes kann es sich insbesondere um einen Gutwickel und/oder einen Schlechtwickel des Analysebands handeln. In der Regel wird jedoch der Schlechtwickel angetrieben. Gemäß der Erfindung ist das zweite Reibelement in axialer Richtung zwischen mindestens zwei ersten Reibelementen eingebettet, beispielsweise indem in axialer Richtung, also parallel zur Achse, zunächst mindestens ein eines Exemplar eines ersten Reibelements, dann mindestens ein zweites Reibelement und anschließend ein zweites Exemplar eines ersten Reibelements, welches gleich oder verschieden zu dem ersten Exemplar ausgestaltet sein kann, angeordnet ist. Auch eine Abfolge mit mehr als zwei Übergängen zwischen einem ersten und einem zweiten Reibelement ist möglich. Die Reibflächen können insbesondere senkrecht zur Achse ausgerichtet sein. Vorzugsweise wirkt mindestens eine axiale Kraftkomponente auf das zweite Reibelement. Die auf das zweite Reibelement wirkende Kraft kann dabei, wie aus den nachfolgenden Ausführungsbeispielen noch näher hervorgeht, rein in axialer Richtung wirken, kann jedoch auch Komponenten in einer radialen Richtung aufweisen.

Das erste Reibelement und/oder das zweite Reibelement können mindestens eine Reibhülse umfassen, insbesondere mindestens eine axial ausgerichtete Reibhülse. Unter einer Reibhülse ist dabei grundsätzlich ein beliebig geformtes Element, beispielsweise ein hülsenförmiges Element, zu verstehen, welches mindestens eine Reibfläche bereitstellt. Unter einer axialen Ausrichtung ist dabei eine Ausrichtung zu verstehen, bei welcher eine Mitnahme durch eine axiale Reibungskraft erfolgt, also eine Reibungskraft, welche im Wesentlichen parallel zur Achse ausgerichtet ist, also mit einem Winkel von maximal 20° zur Achse, vorzugsweise maximal 10° und besonders bevorzugt maximal 5°. Die axiale Ausrichtung schließt jedoch eine tangentiale Mitnahme des Schlechtwickels nicht aus. Auch eine andere Ausgestaltung ist grundsätzlich möglich.

Die Reibhülse kann beispielsweise zwischen das Analyseband und den Antrieb geschaltet sein, beispielsweise zwischen einen Schlechtwickel und einen Körper der Rutschkupplung. Die Reibhülse kann beispielsweise als Zylinderring oder Zylinderhülse ausgestaltet sein. Die Reibhülse kann beispielsweise mit dem Schlechtwickel verbunden sein oder einstückig mit dem Schlechtwickel ausgebildet sein. Alternativ oder zusätzlich kann die Reibhülse auch mit dem Körper der Rutschkupplung einstückig ausgebildet sein oder mit diesem verbunden sein. Die Reibhülse kann vorzugsweise auch in einer axialen Richtung beweglich gelagert sein, beispielsweise beweglich auf einem Körper der Rutschkupplung. Die Reibhülse kann einteilig oder auch mehrteilig ausgestaltet sein. Die Reibhülse kann beispielsweise mit einem oder mehreren Mitnehmerstiften mit der Achse und/oder einem Körper der Rutschkupplung und/oder dem Wickel, beispielsweise dem Schlechtwickel, verbunden sein.

Mit der vorgeschlagenen Ausgestaltung, insbesondere unter Verwendung einer oder mehrerer Reibhülsen, ist eine besonders bauraumsparende Herstellung einer Rutschkupplung möglich. Insbesondere lässt sich auch im Vergleich zu aus dem Stand der Technik bekannten reibschlüssigen Rutschkupplungen die Teilezahl erheblich reduzieren.

Das erste Reibelement und/oder das zweite Reibelement können in einer Ausführungsform der Erfindung insbesondere mindestens eine kegelförmige Reibfläche umfassen. Dabei ist es besonders günstig, wenn die Reibhülse, wenn eine derartige Reibhülse vorgesehen ist, mindestens eine gegenüber der Achse verkippte Reibfläche umfasst, wobei das erste Reibelement mindestens eine konzentrisch zu der Achse angeordnete kegelförmige Reibfläche umfasst, welche mit der verkippten Reibfläche reibschlüssig zusammen wirkt. Auf diese Weise kann beispielsweise die kegelförmige Reibfläche axial umlaufend ausgestaltet sein, wohingegen die Reibhülse punktuell, in einem größeren Winkelbereich oder ebenfalls vollständig umlaufend auf dieser kegelförmigen Reibfläche aufliegt und den genannten Reibschluss bewirkt.

Die kegelförmige Reibfläche kann dabei auch mehrteilig ausgestaltet sein. Beispielsweise kann die kegelförmige Reibfläche eine auf einer ersten Seite der Reibhülse angeordnete erste kegelförmige Fläche und einer auf einer gegenüberliegenden Seite der Reibhülse angeordnete zweite kegelförmige Fläche umfassen.

Die kegelförmigen Reibflächen und/oder Reibhülsen können dabei auch in einer Schnittebene senkrecht zur Achse ineinander verzahnt ausgestaltet sein. So kann beispielsweise in einer Schnittebene senkrecht zur Achse, ausgehend von der Achse, zunächst mindestens eine dem Wickel zugeordnete zweite Reibhülse mit mindestens einer nach außen weisenden Reibfläche angeordnet sein, gefolgt von mindestens einer der Achse oder einem Körper der Rutschkupplung zugeordneten ersten Reibhülse, mit mindestens einer der Achse zuweisenden ersten Reibfläche, welche mit der zweiten Reibfläche zusammenwirkt. Die erste Reibfläche kann dann beispielsweise derart ausgestaltet sein, dass diese die zweite Reibfläche zumindest abschnittsweise, vorzugsweise vollständig, umschließt. So können die Reibflächen ineinander eingreifen oder ineinander verzahnt sein, was die Stabilität der Rutschkupplung noch erhöht.

Wird eine Ausgestaltung mit der kegelförmigen Fläche verwendet, so wirken nicht ausschließlich Anpresskräfte zwischen dem ersten Reibelement und dem zweiten Reibelement in axialer Richtung, sondern auch in einer radialen Richtung. Daneben sind auch Ausgestaltungen möglich, bei welchen ausschließlich axiale Anpresskräfte wirken. So kann beispielsweise die Reibhülse auf einer ersten Seite mit einer Kraft in axialer Richtung beaufschlagt werden, und auf einer zweiten Seite mit einer Gegenkraft, ebenfalls in rein axialer Richtung. Allgemein sind Ausgestaltungen denkbar, bei denen rein axiale Kräfte, rein radiale Kräfte oder Kräfte mit Komponenten sowohl in axialer als auch in radialer Richtung realisiert werden. Ein Ausführungsbeispiel der letzteren Variante ist die beschriebene Ausgestaltung mit der mindestens einen kegelförmigen Reibfläche.

Wie oben dargestellt, sind zwischen dem ersten Reibelement und dem zweiten Reibelement ein oder mehrere Zwischenelemente vorgesehen, die den Reibschluss bewirken können. In diesem Fall liegt das erste Reibelement nicht unmittelbar auf dem zweiten Reibelement auf, sondern es erfolgt eine Aufteilung des Reibschlusses in mehrere Werkstoffpaarungen, nämlich mindestens eine Werkstoffpaarung des mindestens einen ersten Reibelements mit dem mindestens einen Zwischenelement und mindestens eine Werkstoffpaarung des mindestens einen Zwischenelements mit dem mindestens einen zweiten Reibelement. Das mindestens eine Zwischenelement, beispielsweise der mindestens eine O-Ring, kann auch eine Federwirkung entfalten oder ausüben, so dass beispielsweise eine Vorspannung zwischen dem ersten Reibelement und dem zweiten Reibelement realisiert wird. Das mindestens eine Zwischenelement, insbesondere der mindestens eine O-Ring, weist zumindest teilweise elastische Eigenschaften auf und/oder ist elastisch verformbar ausgestaltet. Das Zwischenelement kann auch zumindest teilweise die Aufgabe eines Federelements mit übernehmen, so dass ein zusätzliches optionales Federelement eingespart werden kann. Ein Federelement kann jedoch optional alternativ oder zusätzlich vorgesehen sein, beispielsweise eine Metallfeder. Ausführungsbeispiele werden unten näher ausgeführt.

Das Zwischenelement kann insbesondere mindestens einen O-Ring umfassen, beziehungsweise als O-Ring ausgestaltet sein. Dementsprechend kann der Reibschluss zwischen dem ersten Reibelement und dem zweiten Reibelement beispielweise über mindestens einen O-Ring erfolgen. Unter einem O-Ring ist dabei ein vorzugsweise flexibles, geschlossenes ringförmiges Element zu verstehen, welches beispielsweise einen runden oder polygonalen Querschnitt aufweisen kann. Der O-Ring kann beispielsweise aus einem Kunststoffwerkstoff hergestellt sein, beispielsweise einem Elastomer oder einem Thermoplast. Beispielsweise kann der O-Ring ganz oder teilweise aus einem Kautschukwerkstoff oder auch einem Fluorelastomer hergestellt sein, beispielsweise aus Viton. Auch andere Werkstoffe sind möglich.

Der Reibschluss zwischen dem ersten Reibelement und dem zweiten Reibelement kann vorzugsweise einstellbar ausgestaltet sein. So kann beispielsweise mindestens ein Einstellelement vorgesehen sein, mittels dessen eine Reibungskraft zwischen dem ersten Reibelement und dem zweiten Reibelement einstellbar ist. Beispielsweise kann auf diese Weise der Übergang zwischen der Haftreibung und der Gleitreibung, welche vorzugsweise den Schaltpunkt der Rutschkupplung definiert, eingestellt werden. Beispielsweise kann mit dieser Einstellung ein Auslieferungszustand des analytischen Testgeräts eingestellt werden. Eine Einstellung durch einen Benutzer des analytischen Testgeräts ist jedoch vorzugsweise nicht gegeben. Das Einstellelement kann beispielsweise mindestens eine Einstellmutter umfassen. Auch andere Ausgestaltungen des Einstellelements sind jedoch grundsätzlich alternativ oder zusätzlich möglich. Weiterhin kann, alternativ oder zusätzlich zu dem Einstellelement, mindestens ein Federelement vorgesehen sein, welches eingerichtet ist, um eine Reibungskraft zwischen dem ersten Reibelement und dem zweiten Reibelement zu beeinflussen. Beispielsweise kann das Federelement eingerichtet sein, um das erste Reibelement auf das zweite Reibelement zu pressen oder umgekehrt. Auf diese Weise kann insbesondere für eine konstante Reibungskraft beziehungsweise für konstante Kraftverhältnisse gesorgt werden, und die Rutschkupplung kann mechanisch robuster ausgestaltet werden.

Die Möglichkeit einer einstellbaren Realisierung des Reibschlusses und insbesondere des maximalen Drehmonets stellt einen entscheidenden Vorteil der vorgeschlagenen Rutschkupplung dar, im Gegensatz beispielsweise zur WO 2009/037341 A1 oder WO 2008/138443 A1. Alternativ kann der Reibschluss jedoch auch mit einer festen Vorgabe vorgegeben ausgestaltet sein. Beispielsweise kann der Reibschluss einmalig geeignet bestimmt werden und dann daraus eine feste Fixierung abgeleitet werden, beispielsweise durch eine relative Ausrichtung einzelner Komponenten mit einer anschließenden Fixierung. Letzteres kann beispielsweise durch einen Schnappschluss, einen Simmerring oder ähnliche Fixierungselemente erfolgen. Alternativ oder zusätzlich können stoffschlüssige Verbindungstechniken zur Fixierung eingesetzt werden, beispielsweise ein Verkleben und/oder ein Verschweißen.

Das Analyseband kann, wie oben dargestellt, beispielsweise mittels eines oder mehrerer Wickel in das analytische Testgerät eingebracht sein. Dieses Einbringen kann auch austauschbar erfolgen, insbesondere mit Hilfe eines Bandmagazins, welches in einem Grundgerät des analytischen Testgeräts aufnehmbar ist. Dabei kann die Transportvorrichtung über das Grundgerät und das Bandmagazin verteilt sein. Dementsprechend kann die Rutschkupplung ganz oder teilweise Bestandteil des Grundgeräts sein oder kann auch ganz oder teilweise Bestandteil des Bandmagazins sein, beispielsweise eines Wickels des Bandmagazins. So kann die mindestens eine Rutschkupplung beispielsweise in dem Grundgerät enthalten sein, in dem Bandmagazin oder verteilt auf das Grundgerät und das Bandmagazin. Es können auch mehrere Rutschkupplungen vorgesehen sein, welche über das Grundgerät und das Bandmagazin verteilt sein können.

Entsprechend dieser Möglichkeiten wird in einem weiteren Aspekt der Erfindung ein Bandmagazin zum Einsatz in einem analytischen Testgerät gemäß einer oder mehreren der vorangehend beschriebenen Ausgestaltungen vorgeschlagen. Das Bandmagazin umfasst mindestens ein Analyseband, wobei auf die obige Beschreibung verwiesen werden kann. Darüber hinaus kann das Bandmagazin beispielsweise einen Gutwinkel oder einen Schlechtwickel umfassen. Die Rutschkupplung kann dabei ganz oder teilweise in dem Bandmagazin enthalten sein. Bezüglich der möglichen Ausgestaltungen der Rutschkupplung kann dabei auf die obige Beschreibung verwiesen werden.

In einem weiteren Aspekt der vorliegenden Erfindung wird allgemein die Verwendung einer Rutschkupplung in einer Transportvorrichtung für ein Analyseband in einem analytischen Testgerät vorgeschlagen. Bei dem analytischen Testgerät kann es sich insbesondere um ein analytisches Testgerät gemäß einer oder mehreren der oben beschriebenen Ausgestaltungen handeln, so dass für mögliche Ausgestaltungen des analytischen Testgeräts auf die obige Beschreibung verwiesen werden kann. Das Analyseband weist eine Mehrzahl von nacheinander einsetzbaren analytischen Hilfsmitteln, beispielsweise Lanzetten und/oder Testfeldern, auf. Die Rutschkupplung weist mindestens ein mit einem Antrieb der Transportvorrichtung koppelbares, insbesondere gekoppeltes, erstes Reibelement und mindestens ein mit dem Analyseband, insbesondere einem Wickel des Analysebands (beispielsweise einem Gutwickel und/oder einem Schlechtwickel) koppelbares, insbesondere gekoppeltes, zweites Reibelement auf. Das erste Reibelement und das zweite Reibelement sind dabei derart reib schlüssig miteinander verbunden, dass das erste Reibelement in einem ersten Drehmomentbereich das zweite Reibelement mitnimmt und in einem zweiten Drehmomentbereich über das zweite Reibelement hinweggleitet. Bezüglich weiterer möglicher Ausgestaltungen kann auf die obige Beschreibung verwiesen werden.

Das analytische Testgerät, das Bandmagazin und die Verwendung weisen gegenüber dem Stand der Technik zahlreiche Vorteile auf. So lässt sich insbesondere die oben definierte Aufgabe realisieren, und es lässt sich eine geräuschlose Transportvorrichtung realisieren. Dies erfordert grundsätzlich keine zusätzlich aufzubringende Kraft, beispielsweise auf Grund eines Vorspannens einer Feder. Zudem lässt sich die Rutschkupplung mit einem minimalem Bauraum realisieren und damit gut in die Transportvorrichtung integrieren. Die Rutschkupplung kann zudem kostengünstig hergestellt werden. Das vorgeschlagene Konzept ist serienfähig einstellbar, beispielsweise mittels des oben beschriebenen Einstellelements. Weiterhin lässt sich erreichen, dass in einem Ruhezustand keine Kraft auf das Analyseband ausgeübt wird.

### Kurze Beschreibung der Figuren

Weitere Einzelheiten und Merkmale der Erfindung ergeben sich aus der nachfolgenden Beschreibung von bevorzugten Ausführungsbeispielen, insbesondere in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche beziehungsweise hinsichtlich ihrer Funktionen einander entsprechende Elemente.

Im Einzelnen zeigen:
- Figur 1: ein erstes Ausführungsbeispiel einer Rutschkupplung für ein analytisches Testgerät in einer Schnittdarstellung;
- Figur 2: ein zweites Ausführungsbeispiel einer Rutschkupplung für ein analytisches Testgerät;
- Figur 3: ein Ausführungsbeispiel eines analytischen Testgeräts; und
- Figur 4: ein drittes Ausführungsbeispiel einer Rutschkupplung für ein analytisches Testgerät.

### Ausführungsbeispiele

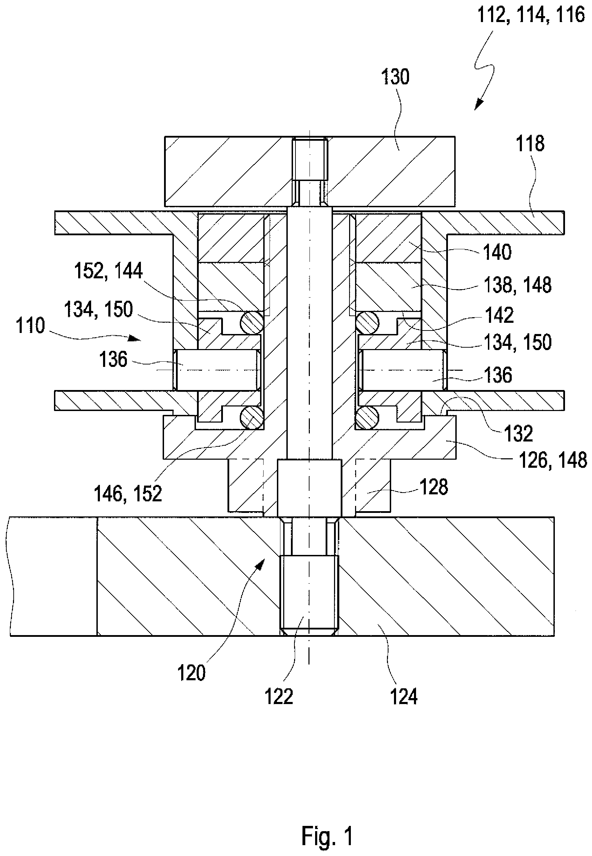

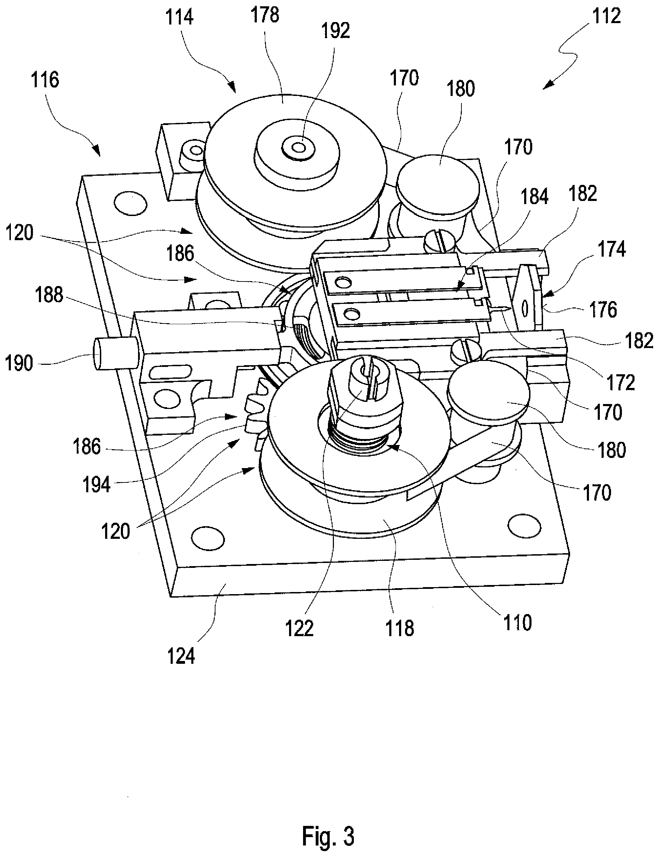

In Figur 1 ist ein erstes Ausführungsbeispiel einer Rutschkupplung 110 in einer Schnittdarstellung von der Seite gezeigt. Diese Rutschkupplung 110 kann in einem analytischen Testgerät 112 eingesetzt werden, welches in Figur 1 lediglich ausschnittsweise gezeigt ist. Beispielsweise kann die Rutschkupplung 110 ganz oder teilweise Bestandteil eines Bandmagazins 114 und/oder eines Grundgeräts 116 des analytischen Testgeräts sein, welches mit dem Bandmagazin 114 zusammenwirkt. Auch andere Ausgestaltungen sind jedoch grundsätzlich möglich. Für Beispiele der sonstigen Ausgestaltung des analytischen Testgeräts 112 kann auf die WO 2008/138443 A1 sowie die WO 2009/037341 A1 verwiesen werden. Die dort dargestellten Testgeräte sind grundsätzlich auch im Rahmen der vorliegenden Erfindung durch Verwendung der dargestellten Rutschkupplung 110 modifizierbar. Auch andere analytische Testgeräte 112 lassen sich jedoch grundsätzlich einsetzen, beispielsweise ein in Figur 3 dargestelltes, einfaches Ausführungsbeispiel eines analytischen Testgeräts 112.

Das analytische Testgerät 112 umfasst in dem dargestellten Ausführungsbeispiel einen Schlechtwickel 118, auf welchen verbrauchte Abschnitte eines Analysebands, beispielsweise eines Lanzettenbandes, aufgewickelt werden. Das Analyseband ist in Figur 1 nicht dargestellt.

Weiterhin umfasst das analytische Testgerät 112 eine in Figur 1 teilweise dargestellte Transportvorrichtung 120 zum Transport des Analysebands, insbesondere zum Weitertakten jeweils eines analytischen Hilfsmittels, beispielsweise einer Lanzette, in eine Anwendungsposition. Die Transportvorrichtung 120 umfasst eine Achse 122, welche beispielsweise in einem Grundgerät montiert sein kann. Im dargestellten Ausführungsbeispiel ist diese Achse 122 in einer Grundplatte 124 montiert.

Auf diese Achse 122 ist im dargestellten Ausführungsbeispiel optional eine Zahnradachse 126 aufgebracht, beispielsweise aufgesteckt. Die Zahnradachse 126 ist dabei hülsenförmig ausgestaltet und weist beispielsweise an ihrem unteren Ende ein Zahnrad 128 auf, welches beispielsweise mittels eines entsprechenden Antriebsmechanismus angetrieben werden kann. Auch eine andere Art des Antriebs ist jedoch grundsätzlich möglich. Die Zahnradachse 126 ist an ihrem oberen Ende durch eine Sicherungsmutter 130, welche beispielsweise auf die Achse 122 aufgesteckt und/oder aufgeschraubt sein kann, gesichert.

Im dargestellten Ausführungsbeispiel ist also eine zweiteilige Ausgestaltung einer Achskonstruktion vorgesehen, nämlich in Form einer Achse 122 und einer separaten Zahnradachse 126. Grundsätzlich wäre jedoch auch eine einteilige Ausgestaltung denkbar, bei welcher die Achse 122 und die Zahnradachse 126 zu einem Bauteil zusammengefasst sind. Die Zahnradachse 126 weist optional in dem dargestellten Ausführungsbeispiel eine Stufe 132 auf. Diese Stufe 132 nimmt den Schlechtwickel 118 auf, welcher somit die Zahnradachse 126 oberhalb der Stufe 132 konzentrisch umgibt. Der Schlechtwickel 118 weist in dem dargestellten Ausführungsbeispiel zwei diametral einander gegenüber liegende Reibhülsen 134 auf, beispielsweise Reibhülsen 134 aus einem Kunststoffmaterial. Diese sind mit dem Schlechtwickel 118, beziehungsweise einem Grundkörper des Schlechtwickels 118 über Mitnehmerstifte 136 verbunden. Grundsätzlich wäre jedoch auch eine einstückige Ausgestaltung der Reibhülsen 134 mit dem Grundkörper des Schlechtwickels 118 möglich. Auch eine andere Anzahl als zwei Reibhülsen 134 wäre denkbar, beispielsweise eine Ausgestaltung mit mehr als zwei Reibhülsen 134 und/oder eine Ausgestaltung mit einer einzigen Reibhülse 134, beispielsweise einer über einen größeren Winkel, bis hin zu 360 C°, umlaufende Reibhülse.

Die Reibhülsen 134 sind im dargestellten Ausführungsbeispiel in axialer Richtung eingebettet zwischen der Stufe 132 der Zahnradachse 126 und einer Einstellmutter 138. Die Einstellmutter 138 kann ihrerseits an ihrem oberen Ende mit einer Kontermutter 140 gesichert sein. Die Einstellmutter 138 und die Kontermutter 140 können beispielsweise auf einem Außengewinde der Zahnradachse 126 aufgeschraubt werden.

Zwischen einer Unterseite 142 der Einstellmutter 138 und den Reibhülsen 134 ist in dem dargestellten Ausführungsbeispiel ein erster O-Ring 144 vorgesehen. In gleicher Weise ist zwischen den Reibhülsen 134 und der Stufe 132 ein zweiter O-Ring 146 vorgesehen. Die Reibhülsen 134 sind dabei derart ausgestaltet, dass diese keine weiteren Reibflächen mit der Zahnradachse 126 bilden. Die Zahnradachse 126 und die Einstellmutter 138, welche auch als Teil der Zahnradachse 126 angesehen werden kann, bilden somit ein erstes Reibelement 148, und die Reibhülse 134 bildet ein zweites Reibelement 150. Zwischen diesen Reibelementen 148, 150 wird ein Reibschluss hergestellt, welcher in einem niedrigen Drehmomentbereich, in welchem eine Haftreibung zwischen diesen Reibelementen 148, 150 noch nicht überwunden ist, ein Reibschluss und damit ein Mitnahmeeffekt bewirkt, wohingegen ab einer Überwindung der Haftreibung und ab Eintreten einer Gleitreibung kein Mitnahmeeffekt mehr auftritt. Ab diesem Grenz-Drehmoment gleitet das erste Reibelement 148 ohne Mitnahmeeffekt über das zweite Reibelement 150 hinweg. Der Reibschluss wird dabei im dargestellten Ausführungsbeispiel durch Zwischenelemente 152 in Form der O-Ringe 144, 146 bewirkt. Grundsätzlich wäre jedoch auch ein direkter Reibschluss möglich.

In dieser ersten dargestellten Variante der Rutschkupplung 110 wird das Drehmoment als auf den Schlechtwickel 118 mittels der O-Ringe 144, 146 übertragen. Beispielsweise kann lediglich eine Anpresskraft in axialer Richtung wirken, welche das erste Reibelement 148 auf das zweite Reibelement 150 presst oder umgekehrt. Diese Anpresskraft kann mittels der Einstellmutter 138 eingestellt werden. Bei der Drehmomentübertragung wird das Zahnrad 128 auf der Zahnradachse 126 gedreht, wodurch die Zahnradachse 126 gedreht wird und reibschlüssig auf die O-Ringe 144, 146 einwirkt. Diese sind vorzugsweise derart angeordnet, dass keine weitere Reibfläche einen Einfluss auf das zu übertragende Drehmoment hat. Durch die Einstellmutter 138 wird die Vorspannung der O-Ringe zwischen dem Zahnrad beziehungsweise der Zahnradachse 126 und den Reibhülsen 134, die den Schlechtwickel 118 mitnehmen, eingestellt. Dazu wird vorzugsweise ein Feingewinde verwendet, um eine möglichst feine Einstellung zu ermöglichen. In der Serienfertigung kann anstelle einer derart feinen Einstellung über ein Feingewinde jedoch grundsätzlich auch eine andere Verbindung verwendet werden, beispielsweise unter Berücksichtigung der Toleranzen eine fixe Schnappverbindung oder eine ähnliche Verbindung, was einen Montage- und Einstellungsaufwand erheblich reduzieren könnte. Auch ein Kostenaufwand ließe sich dadurch reduzieren.

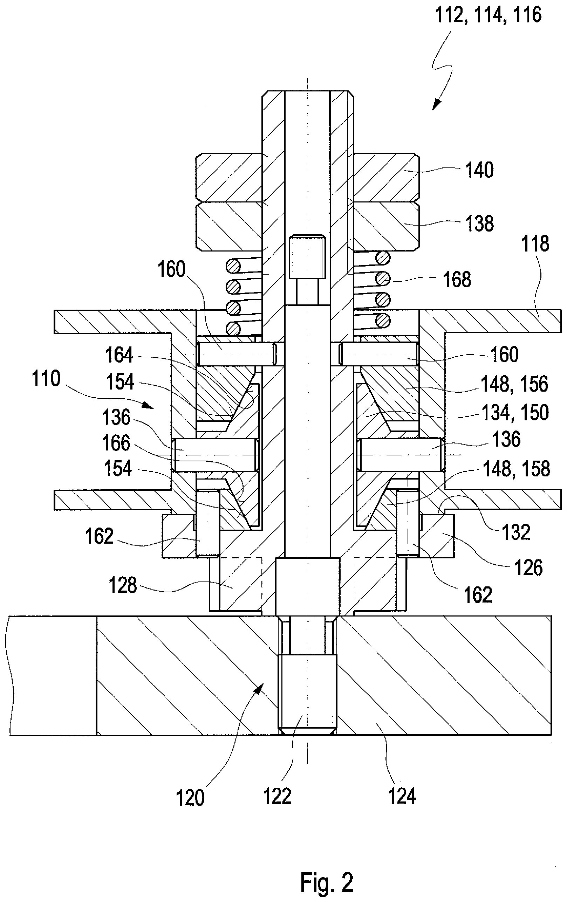

Das dargestellte Ausführungsbeispiel der Rutschkupplung 110 ist, wie auch das in Figur 2 dargestellte Ausführungsbeispiel, im Rahmen der Erfindung grundsätzlich auch modifizierbar. So kann beispielsweise anstelle eines Schlechtwickels 118 auch ein anderes Element angetrieben werden, beispielsweise ein Gutwickel, was jedoch weniger bevorzugt ist.

Die dargestellten Komponenten können beispielsweise vollständig oder teilweise aus metallischen und/oder Kunststoff-Werkstoffen hergestellt werden. Dabei kann, wie oben ausgeführt, beispielsweise eine Ausgestaltung gewählt werden, bei der an jeder Kontaktstelle zwischen den Reibelementen 148, 150 gleiche Werkstoffpaarungen vorliegen. So könnten beispielsweise die Einstellmutter 138 und die Zahnradachse 126, beziehungsweise die Stufe 132 aus identischen Materialien gefertigt werden, so dass an jeder Reibstelle gleiche Werkstoffpaarungen auftreten, beispielsweise an allen Grenzflächen zwischen der Einstellmutter, beziehungsweise der Stufe 132 und den O-Ringen 144, 146 eine erste Werkstoffpaarung und an allen Grenzflächen zwischen den O-Ringen 144, 146 und den Reibhülsen 134 eine zweite Werkstoffpaarung. Auch eine andere Ausgestaltung ist jedoch grundsätzlich möglich, also eine Ausgestaltung mit unterschiedlichen Materialien beziehungsweise unterschiedlichen Werkstoffpaaren.

Weiterhin wird nochmals darauf hingewiesen, dass die Rutschkupplung 110 auch ganz oder teilweise in den Wickel, beispielsweise dem Schlechtwickel 118 integriert sein kann. Dementsprechend kann die Rutschkupplung im Idealfall auch beispielsweise vollständig oder teilweise Bestandteil eines Bandmagazins 114 sein, welches austauschbar in einem Grundgerät 116 in das analytischen Testgerät 112 aufgenommen werden kann.

In Figur 2 ist ein zweites Ausführungsbeispiel einer Rutschkupplung 110 in einer zur Figur 1 analogen Darstellung gezeigt. Wiederum ist diese in einem analytischen Testgerät 112 aufgenommen, beispielsweise ganz oder teilweise in einem Bandmagazin 114, beispielsweise einem Schlechtwickel 118 eines Bandmagazins 114. Für Ausführungsbeispiele möglicher analytischer Testgeräte 112, in denen die Rutschkupplung 110 gemäß Figur 2 zum Einsatz kommen kann, kann wiederum beispielsweise auf die WO 2008/138443 A1 oder die WO 2009/037341 A1 verwiesen werden oder auf das analytischen Testgerät 112 gemäß Figur 3. Auch andere Ausgestaltungen sind jedoch grundsätzlich möglich.

Das analytische Testgerät 112 weist wiederum auch eine Transportvorrichtung 120 zum Transport eines analytischen Testbandes (in Figur 2 wieder nicht dargestellt) auf. Dieses umfasst wiederum eine Achskonstruktion, welche im dargestellten Beispiel wieder zweiteilig ausgestaltet ist. Analog zu Figur 1 ist jedoch wiederum auch eine andere Konstruktion möglich. Bei dieser zweiteiligen Achskonstruktion ist wiederum eine Achse 122 in einer Grundplatte 124 oder einem anderen Trägerelement aufgenommen. Auf diese Achse 122 aufgebracht ist eine Zahnradachse 126, welche an ihrem unteren Ende wiederum ein Zahnrad 128 aufweist und/oder eine andere Art von Koppelelement, um die Zahnradachse 126 an einen Antrieb zu koppeln.

Die Zahnradachse 126 weist wiederum eine Stufe 132 auf, welche den Schlechtwickel 118 aufnimmt. Wie auch im Ausführungsbeispiel gemäß Figur eins liegt jedoch vorzugsweise kein unmittelbarer Kontakt zwischen der Stufe 132 und dem Schlechtwickel 118 vor. Der Schlechtwickel 118 und die Zahnradachse 126 sind wiederum durch eine Rutschkupplung 110 miteinander reibschlüssig verbunden. Die Rutschkupplung 110 weist dabei wiederum ein erstes Reibelement 148 auf, welches mit der Zahnradachse 126 verbunden ist und/oder Bestandteil der Zahnradachse 126 ist und damit mit dem Antrieb verbindbar ist, sowie ein zweites Reibelement 150, welches mit dem Wickel, in diesem Fall dem Schlechtwickel 118, verbunden beziehungsweise verbindbar ist.

Wiederum ist das zweite Reibelement 150 beispielsweise in Form einer oder mehrerer Reibhülsen 134 ausgebildet, beispielsweise wiederum zweier diametral gegenüberliegender Reibhülsen 134. Diese können beispielsweise wiederum über Mitnehmerstifte 136 mit dem Schlechtwickel 118 verbunden sein und/oder auch fester Bestandteil des Schlechtwickels 118 sein. Auch andere Verbindungen mit dem Schlechtwickel 118 sind denkbar.

Im Unterschied zur Ausgestaltung gemäß Figur 1 sind die Reibhülsen 134 im dargestellten Ausführungsbeispiel kegelförmig ausgestaltet und weisen gegenüber der Achse 122 verkippte Reibflächen 154 auf.

Das erste Reibelement 148 seinerseits ist in diesem Ausführungsbeispiel vorzugsweise zweiteilig ausgestaltet und weist einen ersten Kegel 156 oberhalb der Reibhülsen 134 und einen zweiten Kegel 158 unterhalb der Reibhülsen 134 auf. Der erste und der zweite Kegel 156 und 158 können beispielsweise wiederum über Mitnehmerstifte 160 und 162 mit der Zahnradachse 126 verbunden sein. Auch eine andere Ausgestaltung ist jedoch grundsätzlich möglich, beispielsweise eine Integration oder feste Verbindung zwischen den Kegeln 156, 158 und der Zahnradachse 126.

Die Kegel 156, 158 stellen ihrerseits zu der Achse 122 verkippte Reibflächen bereit, nämlich eine erste kegelförmige Fläche 164 und eine zweite kegelförmige Fläche 166, welche mit den verkippten Reibflächen 154 der Reibhülsen 134 zusammenwirken. Wiederum erfolgt dabei bis zu einem Grenzdrehmoment oder einem Grenzdrehmomentbereich, in welchem die Haftreibung wirkt, ein Mitnahmeeffekt zwischen dem ersten Reibelement 158 und dem zweiten Reibelement 150, wohingegen bei Überschreiten dieses Grenzdrehmoments das erste Reibelement 158 über das zweite Reibelement 150 hinweggleitet.

Wiederum kann eine Einstellbarkeit der Anpresskraft vorgesehen sein, mit welcher das erste Reibelement 148 auf das zweite Reibelement 150 gepresst wird und/oder umgekehrt. Zu diesem Zweck ist in dem dargestellten Ausführungsbeispiels wiederum eine Einstellmutter 138 vorgesehen, welche beispielsweise wiederum über ein Feingewinde auf die Zahnradachse 126 aufgeschraubt ist. Zur Sicherung der Einstellmutter kann wiederum auch eine Kontermutter 140 vorgesehen sein.

In dem dargestellten Ausführungsbeispiel kann die Einstellmutter 138 beispielsweise unmittelbar auf den ersten Kegel 156 einwirken. Dargestellt ist jedoch eine Konstruktion, bei welcher die Einstellmutter 138 über ein Federelement 168 auf den ersten Kegel 156 einwirkt. Hierdurch kann eine mechanisch robuste Konstruktion gewährleistet werden. Beispielsweise kann der erste Kegel 156 mit vergrößerten Bohrungen für die Mitnehmerstifte 160 versehen sein, so dass die Mitnehmerstifte 160 in diesen Bohrungen ein gewisses Spiel aufweisen, so dass das Federelement 168 eine geringfügige Lageveränderung des ersten Kegels 156 in axialer Richtung bewirken kann.

Wiederum sind beispielsweise auch in dem dargestellten Ausführungsbeispiel gemäß Figur 2 sämtliche Reibflächen mit gleichen Werkstoffpaarungen versehen. So können beispielsweise der erste Kegel 156 und der zweite Kegel 158 aus identischem Material hergestellt sein, so dass ein Übergang zwischen dem ersten Kegel 156 und den Reibhülsen 134 und am Übergang zwischen dem zweiten Kegel 156 und den Reibhülsen 134 die gleichen Werkstoffpaare beziehungsweise Reibpaare vorliegen. Auch eine andere Ausgestaltung ist jedoch wiederum möglich, also eine Ausgestaltung mit unterschiedlichen Werkstoffpaaren.

In dem zweiten Ausführungsbeispiel gemäß Figur 2 wird das Drehmoment auf den Schlechtwickel 118 mittels der kegelförmigen Flächen 164, 166 übertragen. Dabei wird das Zahnrad 128 auf der Zahnradachse 126 gedreht und wirkt somit indirekt auf diese kegelförmigen Flächen 164, 166 ein. Diese sind vorzugsweise wiederum derart angeordnet, dass keine weiteren Reibflächen Einfluss auf das zu übertragende Drehmoment haben. Durch die Einstellmutter 138, die gegen das Federelement 168, beispielsweise eine Spiralfeder, drückt, wird die Vorspannung der Kegel 156, 158 zwischen Zahnrad 128 und Reibhülsen 134 eingestellt. Dazu wird bevorzugt wiederum ein Feingewinde verwendet, um mittels der Einstellmutter 138 eine feinere Einstellung zu ermöglichen. Wiederum ist in einer Serienfertigung auch vorstellbar, unter Berücksichtigung von Toleranzen eine andere Art der Verbindung zu realisieren, beispielsweise eine Schnappverbindung, anstelle einer Einstellmutter, was wiederum montage- und kostenfreundlicher wäre.

In dem dargestellten Beispiel wirkt eine Anpresskraft zwischen dem ersten Reibelement 148 und dem zweiten Reibelement 150 nicht ausschließlich in axialer Richtung, sondern senkrecht zu den kegelförmigen Flächen 164, 166 mit einer radialen Komponente.

Wie auch die Darstellung in Figur 1, ist auch die Darstellung des zweiten Ausführungsbeispiels in Figur 2 lediglich als Prinzipdarstellung zu verstehen. Weitere Ausführungsmöglichkeiten, beispielsweise Abwandlungen der in Figur 2 dargestellten Ausführungsform, sind im Rahmen der vorliegenden Erfindung denkbar. Wiederum kann die dargestellte Rutschkupplung 110 im Idealfall auch vollständig in das Bandmagazin 114, beispielsweise in den Schlechtwickel 118 integriert werden. Auch eine Ausgestaltung vollständig ohne Bandmagazin 114 ist jedoch denkbar, beispielsweise eine Ausführung, bei welcher das Analyseband ohne Verwendung eines Bandmagazins in das analytische Testgerät 112 eingebracht wird, beispielsweise ausschließlich mittels eines oder mehrerer Wickel und/oder als einzelnes Analyseband. Für den Antrieb des analytischen Testgeräts 112, also Komponenten, welche in Figur 2 nicht dargestellt sind, kann exemplarisch wiederum auf die WO 2008/138443 A1 oder die WO 2009/037341 A1 oder das Ausführungsbeispiel gemäß Figur 3 verwiesen werden. Beispielsweise kann ein automatischer Antrieb vorgesehen sein, beispielsweise mittels eines Elektromotors und/oder einer Handbedienung, beispielsweise mittels eines manuell betätigbaren Aktuators. Verschiedene Ausgestaltungen besonderer Art sind denkbar.

In Figur 3 ist in stark schematisierter perspektivischer Darstellung ein Ausführungsbeispiel eines analytischen Testgeräts 112 dargestellt. Das analytische Testgerät 112 umfasst ein Analyseband 170, welches mittels einer Transportvorrichtung 120 in dem analytischen Testgerät 112 transportiert wird. Das Analyseband 170 kann beispielsweise eine Mehrzahl von analytischen Hilfsmitteln 172 umfassen, welche beispielsweise in einer Bandrichtung hintereinander auf dem Analyseband 170 oder einem Trägerband des Analysebands 170 angeordnet sind und welche beispielsweise nacheinander in einer Applikationsposition 174 des analytischen Testgeräts 112 eingesetzt werden können. In dieser Applikationsposition 174 kann beispielsweise eine Auflagefläche 176 zur Auflage auf eine Hautoberfläche eines Benutzers vorgesehen sein. In Figur 3 ist lediglich ein analytisches Hilfsmittel 172 angedeutet.

Die Transportvorrichtung 120 umfasst einen Gutwickel 178, auf welchem Abschnitte des Analysebands 170 mit noch unbenutzten analytischen Hilfsmitteln 172 aufgenommen sind, sowie einen Schlechtwickel 118, auf welchem Abschnitte des Analysebands 170 mit benutzten analytischen Hilfsmitteln 172, beispielsweise Lanzetten, aufgenommen sind. Das Analyseband 170 kann beispielsweise mittels Umlenkungen 180, insbesondere Umlenkrollen, zu der Applikationsposition 174 geführt werden. Dabei drehen sich beispielsweise in der Darstellung gemäß Figur 3 der Gutwickel 178 und der Schlechtwickel 118 im Uhrzeigersinn. Vor Erreichen der Applikationsposition 174 kann das Analyseband 170 durch eine Bandführung 182 geführt werden, beispielsweise derart, dass die analytischen Hilfsmittel 172 von einer vertikalen Ebene in eine horizontale Ebene, beispielsweise parallel zu einer Grundplatte 124, umgelenkt werden. Bei anderen Arten analytischer Hilfsmittel 172, beispielsweise Testelementen, kann auf die eine derartige Umlenkung verzichtet werden.

In der Applikationsposition 174 werden die analytischen Hilfsmittel 172 beispielsweise von einem Greifer 184 oder Greifersystem ergriffen und bestimmungsgemäß verwendet. Beispielsweise kann eine Probenaufnahmebewegung durchgeführt werden und/oder eine Lanzettenbewegung. Bei letzterer kann beispielsweise eine Lanzette eine Öffnung in der Auflagefläche 176 durchstoßen und eine Haut eines Benutzers perforieren. Die Bewegung des Greifers 184 oder Greifersystems kann beispielsweise auf einem Schlitten erfolgen und kann beispielsweise durch einen Antrieb, hier dargestellt durch eine Antriebseinheit 186, angetrieben werden. Die Antriebseinheit 186 ist in Figur 3 lediglich angedeutet und kann beispielsweise ähnlich den in WO 2008/138443 A1 oder die WO 2009/037341 A1 dargestellten Antriebsmechanismen ausgestaltet werden. Die Antriebseinheit 186 kann beispielsweise ein Federelement 188 und/oder eine andere Art eines Energiespeichers umfassen. Beispielsweise kann er dieses Federelement 188 eine Spiralfeder umfassen, welche ein Rad der Antriebseinheit 186 zu einer Drehbewegung antreibt, wodurch beispielsweise der Greifer 184 angetrieben wird, insbesondere zu einer linearen Bewegung in Richtung der Auflagefläche 176. Die Antriebseinheit 186 kann insbesondere durch einen Auslöser 190 ausgelöst werden, beispielsweise einen Auslöser 190, welcher das Rad der Antriebseinheit 186 freigibt.

Der Gutwickel 178 und der Schlechtwickel 118 können jeweils auf Achsen 192 bzw. 122 gelagert seien. Die Transportvorrichtung 120 bzw. deren Antrieb 186 können beispielsweise ein Zahnrad 194 umfassen, welches beispielsweise ebenfalls von dem Federelement 188 und/oder einem anderen Teil der Antriebseinheit 186 angetrieben werden kann und welches beispielsweise mit einem (in Figur 3 nicht erkennbaren) Zahnrad 128 der Achse 122 des Schlechtwickels 118 zusammenwirken kann, um diese anzutreiben.

Die Achse 122 des Schlechtwickels 118 umfasst in dem dargestellten Ausführungsbeispiel eine Rutschkupplung 110. Diese Rutschkupplung 110 kann beispielsweise analog zu dem in Figur 2 dargestellten Ausführungsbeispiel ausgestaltet sein. Auch andere Ausgestaltungen sind jedoch grundsätzlich möglich. Die Rutschkupplung 110 bietet die oben genannten Vorteile und gleicht beispielsweise Drehmomentsänderungen aufgrund eines sicher ändernden Aufwicklungsgrades auf dem Schlechtwickel 118 aus und verhindert beispielsweise Beschädigungen des Analysebands 170 und/oder des Greifers 184.

Es wird darauf hingewiesen, dass das analytische Testgerät 112 in Figur 3 stark schematisiert gezeigt ist. Ein mögliches Gehäuse ist nicht dargestellt. Weiterhin ist eine Trennung zwischen einem Grundgerät 116 und einem Bandmagazin 114, welche optional vorhanden sein kann, nicht dargestellt. Zahlreiche andere Ausgestaltungen des analytischen Testgeräts 112 unter Verwendung einer oder mehrerer Rutschkupplungen 110 sind möglich.

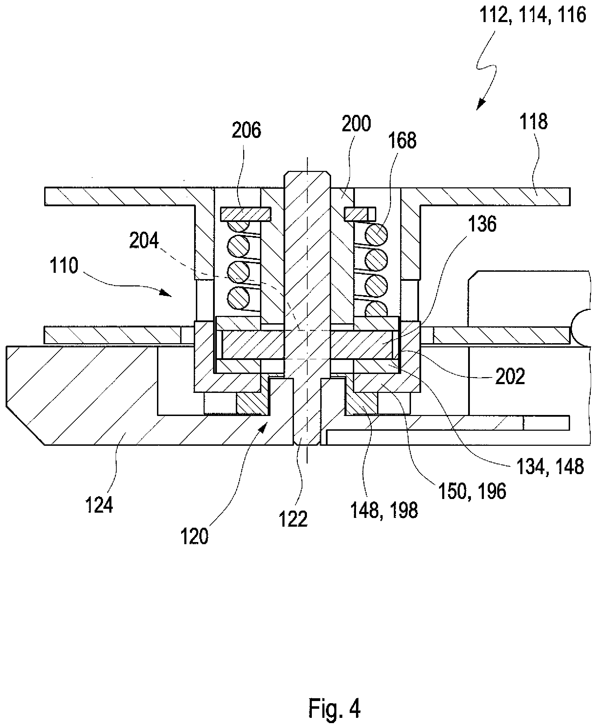

In Figur 4 ist ein weiteres Ausführungsbeispiel eines erfindungsgemäßen analytischen Testgeräts 112 mit einer Rutschkupplung 110 dargestellt. Das Ausführungsbeispiel entspricht in weiten Teilen dem Ausführungsbeispiel gemäß Figur 1, sodass weitgehend auf diese Figur verwiesen werden kann. Wiederum erfolgt in diesem Ausführungsbeispiel die Mitnahme eines Schlechtwickels vorzugsweise aufgrund rein axial, also parallel zur Achse wirkender Reibungskräfte zwischen einem ersten Reibelement 148 und einem zweiten Reibelement 150. Auch eine leichte Verkippung der Reibungskräfte relativ zur Achse 122 ist jedoch grundsätzlich möglich, vorzugsweise eine Verkippung um nicht mehr als 10 Grad.

Das erste Reibelement 148 ist in dem dargestellten Ausführungsbeispiel wiederum zweiteilig ausgestaltet, ähnlich zu dem Ausführungsbeispiel gemäß Figur 1. Dabei ist das zweite Reibelement 150, welches mit dem Schlechtwickel 118 verbunden ist, als sich von dem eigentlichen Schlechtwickel 118 aus nach innen, zur Achse 122 hin erstreckender scheibenförmiger Fortsatz 196 ausgebildet, welcher einstückig oder auch in Form einer Einwegverbindung oder einer lösbaren Verbindung mit dem übrigen Schlechtwickel 118 verbunden sein kann. Dieser scheibenförmige Fortsatz 196 ist eingebettet zwischen einen oberen Teil des ersten Reibelements 148, der in diesem Fall als Reibhülse 134 ausgebildet ist und einen unteren Teil des ersten Reibelements 148, welcher in diesem Fall als nach außen vorspringende Stufe 198 des auf die Achse 122 aufgebrachten oder fest mit der Achse 122 verbundenen Körpers 200 der Rutschkupplung 110 ausgebildet ist. Im Gegensatz zum Ausführungsbeispiel gemäß Figur 1 ist die Reibhülse 134 also in diesem Ausführungsbeispiel nicht Anteil des zweiten Reibelements 150, sondern des ersten Reibelements 148. Auch eine Mischform ist denkbar, mit einer oder mehreren Reibhülsen 134, die dem ersten Reibelement zugeordnet sind, und einer oder mehreren Reibhülsen 134, die dem zweiten Reibelement 150 zugeordnet sind. Die Reibhülse 134 in dem in Figur 4 dargestellten Ausführungsbeispiel ist als Zylinderring ausgebildet, mit einem Innendurchmesser, welcher derart ausgestaltet ist, dass der Zylinderring über den Körper 200 der Rutschkupplung 110 geschoben werden kann, vorzugsweise passgenau oder mit einem Spiel von nicht mehr als einigen Zehntel Millimetern. In radialer Richtung sind in dem Zylinderring der Reibhülse 134, eine, zwei oder mehrere Bohrungen 202 aufgenommen, durch welche hindurch sich die Mitnehmerstifte 136 oder der Mitnehmerstift 136 erstreckt. Der Mitnehmerstift 136 ist seinerseits in dem in Figur 4 dargestellten Ausführungsbeispiel exemplarisch als ein einzelner Mitnehmerstift 136 ausgestaltet, welcher sich durch eine weitere Bohrung 204 in der Achse 122 hindurch erstreckt. Diese Bohrung 204 ist vorzugsweise als Langlochbohrung ausgestaltet und weist in axialer Richtung eine den Durchmesser des Mitnehmerstifts 136 überschreitende Abmessung auf. Senkrecht zur Achse ist die Abmessung der Bohrung 204 jedoch vorzugsweise der Abmessung des Mitnehmerstifts 136 angepasst. Dementsprechend weist der Mitnehmerstift 136 in axialer Richtung ein Spiel auf, wohingegen senkrecht zur Achse vorzugsweise kein Spiel vorliegt.

Die Reibhülse 134 ist dementsprechend in axialer Richtung bewegbar auf dem Körper 200 der Rutschkupplung 110 gelagert. Wiederum ist ein Federelement 168 vorgesehen, welches in diesem Fall die Reibhülse 134 mit einer in Figur 4 nach unten, als hin zum scheibenförmigen Fortsatz 196 gerichteten Federkraft belastet. An ihrem gegenüber liegenden Ende ist das Federelement 168 gegengelagert, beispielsweise, wie in Figur 4 dargestellt, mittels eines Sicherungsrings 206. Das Federelement 168 ist in dem dargestellten Ausführungsbeispiel als Spiralfeder ausgestaltet. Auch andere Ausgestaltungen des Federelements 168 sowie des Gegenlagers sind denkbar. Das Federelement 168 presst die Reibhülse 134 gegen den scheibenförmigen Fortsatz 196, sodass zwischen diesem scheibenförmigen Fortsatz 196 und der Reibhülse 134 sowie der Stufe 198 eine Reibkraft entsteht. Bei einer Drehung der Achse 122 wird dementsprechend der Schlechtwickel 118 mitgenommen, sofern ein Gegen-Drehmoment nicht das durch die Reibung zwischen den Reibelementen 148, 150 ausgeübte Mitnehmerdrehmoment überschreitet.

Auch Abwandlungen der in Figur 4 dargestellten Ausgestaltung sind möglich. So kann die Stufe 198 des ersten Reibelements 148 auch durch eine andere Art von Vorsprung ersetzt werden, welcher nicht notwendigerweise fest mit dem Körper 200 verbunden sein muss. So kann anstelle einer einstückigen Ausgestaltung der Stufe 198 mit dem Körper 200 auch beispielsweise eine lösbare Verbindung oder eine einmal herstellbare Verbindung eines Vorsprungs mit dem Körper 200 eingesetzt werden. Weiterhin ist der in Figur 4 gezeigte Aufbau auch umkehrbar, sodass beispielsweise auch die Reibhülse 134 als unterer Teil des ersten Reibelements 148 ausgestaltet sein kann, und die Stufe 198 als oberer Teil des ersten Reibelements 148. Weiterhin kann das Federelement 168 auch beispielsweise durch ein Einstellelement ergänzt und/oder ersetzt werden, beispielsweise analog zur Ausgestaltung in Figur 1. Auch dies kann beispielsweise mit einer in axialer Richtung auf dem Körper 200 verschiebbaren Reibhülse 134 erfolgen, analog zur Ausgestaltung in Figur 4. Dementsprechend können die Ausgestaltungen in den Figuren 1 und 4 auch kombiniert werden.

In dem in Figur 4 dargestellten Ausführungsbeispiel ist das erste Reibelement 148 zweiteilig oder allgemein mehrteilig (wobei auch mehr als zwei Teile vorgesehen sein können) ausgestaltet. Dies ist jedoch nicht notwendigerweise der Fall. Alternativ oder zusätzlich kann auch das zweite Reibelement 150 zwei- oder mehrteilig ausgestaltet sein, sodass beispielsweise dieses mindestens zwei Teile aufweisen kann, zwischen denen mindestens ein Teil des ersten Reibelements 148 angeordnet ist. Verschiedene Ausgestaltungen sind denkbar.

**Bezugszeichenliste**

| | | | |
|---|---|---|---|
| 110 | Rutschkupplung | 160 | Mitnehmerstifte |
| 112 | analytisches Testgerät | 162 | Mitnehmerstifte |
| 114 | Bandmagazin | 164 | erste kegelförmige Fläche |
| 116 | Grundgerät | 166 | zweite Kegelförmige Fläche |
| 118 | Schlechtwickel | 168 | Federelement |
| 120 | Transportvorrichtung | 170 | Analyseband |
| 122 | Achse | 172 | Analytische Hilfsmittel |
| 124 | Grundplatte | 174 | Applikationsposition |
| 126 | Zahnradachse | 176 | Auflagefläche |
| 128 | Zahnrad | 178 | Gutwickel |
| 130 | Sicherungsmutter | 180 | Umlenkung |
| 132 | Stufe | 182 | Bandführung |
| 134 | Reibhülse | 184 | Greifer |
| 136 | Mitnehmerstifte | 186 | Antriebseinheit |
| 138 | Einstellmutter | 188 | Federelement |
| 140 | Kontermutter | 190 | Auslöser |
| 142 | Unterseite | 192 | Achse |
| 144 | erster O-Ring | 194 | Zahnrad |
| 146 | zweiter O-Ring | 196 | scheibenförmiger Fortsatz |
| 148 | erstes Reibelement | 198 | Stufe |
| 150 | zweites Reibelement | 200 | Körper |
| 152 | Zwischenelemente | 202 | Bohrung |
| 154 | verkippte Reibfläche | 204 | Bohrung |
| 156 | erster Kegel | 206 | Sicherungsring |
| 158 | zweiter Kegel | | |

## Patentansprüche

1. Analytisches Testgerät (112), aufweisend mindestens eine Transportvorrichtung (120) für ein Analyseband (170), insbesondere ein Lanzettenband, wobei das Analyseband (170) eine Mehrzahl von nacheinander einsetzbaren analytischen Hilfsmitteln (172) umfasst, wobei die Transportvorrichtung (120) mindestens eine Rutschkupplung (110) umfasst, wobei die Rutschkupplung (110) mindestens ein mit einem Antrieb (186) koppelbares erstes Reibelement (148) und mindestens ein mit dem Analyseband (170), insbesondere einem Wickel (118) des Analysebands (170), koppelbares zweites Reibelement (150) aufweist, wobei das erste Reibelement (148) und das zweite Reibelement (150) derart reibschlüssig verbunden sind, dass das erste Reibelement (148) in einem ersten Drehmomentbereich das zweite Reibelement (150) mitnimmt und in einem zweiten Drehmomentbereich über das zweite Reibelement (150) hinweggleitet, wobei die Rutschkupplung (110) zwischen einer angetriebenen Achse (122, 126) und einem die Achse (122, 126) umschließenden Element, insbesondere einem Wickel (118) des Analysebands (170), angeordnet ist, **dadurch gekennzeichnet, dass** zwischen dem ersten Reibelement (148) und dem zweiten Reibelement (150) mindestens ein Zwischenelement vorgesehen ist, wobei das Zwischenelement elastische Eigenschaften aufweist, wobei das zweite Reibelement (150) in axialer Richtung zwischen zwei ersten Reibelementen (148) eingebettet ist, wobei mindestens eine axiale Kraftkomponente auf das zweite Reibelement (150) wirkt.

2. Analytisches Testgerät (112) nach dem vorhergehenden Anspruch, wobei ein Übergang zwischen dem ersten Drehmomentbereich und dem zweiten Drehmomentbereich durch einen Übergang zwischen einer Haftreibung und einer Gleitreibung zwischen dem ersten Reibelement (148) und dem zweiten Reibelement (150) vorgegeben ist.

3. Analytisches Testgerät (112) nach einem der vorhergehenden Ansprüche, wobei mindestens eines der Reibelemente (148, 150) mindestens eine Reibhülse (134) umfasst.

4. Analytisches Testgerät (112) nach einem der vorhergehenden Ansprüche, wobei das erste Reibelement (148) und/oder das zweite Reibelement (150) mindestens eine kegelförmige Reibfläche (164, 166) umfasst.

5. Analytisches Testgerät (112) nach den beiden vorhergehenden Ansprüchen, wobei die Reibhülse (134) mindestens eine gegenüber der Achse (122, 126) verkippte Reibfläche (154) umfasst, wobei das erste Reibelement (148) mindestens eine konzentrisch zu der Achse (122, 126) angeordnete kegelförmige Reibfläche (164, 166) umfasst, welche mit der verkippten Reibfläche (154) reibschlüssig zusammenwirkt.

6. Analytisches Testgerät (112) nach dem vorhergehenden Anspruch, wobei die kegelförmige Reibfläche (164, 166) eine auf einer ersten Seite der Reibhülse (134) angeordnete erste kegelförmige Fläche (164) und eine auf einer gegenüberliegenden Seite der Reibhülse (134) angeordnete zweite kegelförmige Fläche (166) umfasst.

7. Analytisches Testgerät (112) nach einem der vorhergehenden Ansprüche, wobei der Reibschluss zwischen dem ersten Reibelement (148) und dem zweiten Reibelement (150) über das mindestens eine Zwischenelement, insbesondere mindestens einen O-Ring (144, 146), erfolgt.

8. Analytisches Testgerät (112) nach einem der vorhergehenden Ansprüche, wobei mindestens ein Einstellelement vorgesehen ist, wobei mittels des Einstellelements eine Reibungskraft zwischen dem ersten Reibelement (148) und dem zweiten Reibelement (150) einstellbar ist.

9. Analytisches Testgerät (112) nach dem vorhergehenden Anspruch, wobei das Einstellelement mindestens eine Einstellmutter (138) umfasst.

10. Analytisches Testgerät (112) nach einem der vorhergehenden Ansprüche, wobei mindestens ein Federelement (168) vorgesehen ist, wobei das Federelement (168) eingerichtet ist, um eine Reibungskraft zwischen dem ersten Reibelement (148) und dem zweiten Reibelement (150) zu beeinflussen.

11. Analytisches Testgerät (112) nach einem der vorhergehenden Ansprüche, wobei an jeder Kontaktstelle zwischen dem ersten Reibelement (148) und dem zweiten Reibelement (150) gleiche Werkstoffpaare vorliegen.

12. Bandmagazin (114) zum Einsatz in einem analytischen Testgerät (112) nach einem der vorhergehenden Ansprüche, wobei das Bandmagazin (114) das mindestens eine Analyseband (170) umfasst, wobei die Rutschkupplung (110) ganz oder teilweise in dem Bandmagazin (114) enthalten ist.

13. Verwendung einer Rutschkupplung (110) in einer Transportvorrichtung (120) für ein Analyseband (170) in einem analytischen Testgerät (112), insbesondere einem analytischen Testgerät (112) nach einem der vorhergehenden, ein analytisches Testgerät (112) betreffenden Ansprüche, wobei das Analyseband (170) eine Mehrzahl von nacheinander einsetzbaren analytischen Hilfsmitteln (172) umfasst, wobei die Rutschkupplung (110) mindestens ein mit einem Antrieb (186) koppelbares erstes Reibelement (148) und mindestens ein mit dem Analyseband (170), insbesondere einem Wickel (118) des Analysebands (170), koppelbares zweites Reibelement (150) aufweist, wobei das erste Reibelement (148) und das zweite Reibelement (150) derart reibschlüssig verbunden sind, dass das erste Reibelement (148) in einem ersten Drehmomentbereich das zweite Reibelement (150) mitnimmt und in einem zweiten Drehmomentbereich über das zweite Reibelement (150) hinweggleitet, wobei die Rutschkupplung (110) zwischen einer angetriebenen Achse (122, 126) und einem die Achse (122, 126) umschließenden Element, insbesondere einem Wickel (118) des Analysebands (170), angeordnet ist, **dadurch gekennzeichnet, dass** zwischen dem ersten Reibelement (148) und dem zweiten Reibelement (150) mindestens ein Zwischenelement vorgesehen ist, wobei das Zwischenelement elastische Eigenschaften aufweist, wobei das zweite Reibelement (150) in axialer Richtung zwischen zwei ersten Reibelementen (148) eingebettet ist, wobei mindestens eine axiale Kraftkomponente auf das zweite Reibelement (150) wirkt.

## Claims

1. Analytic test instrument (112), having at least one transport device (120) for an analysis tape (170), in particular a lancet tape, wherein the analysis tape (170) comprises a plurality of analytic aids (172) that can be inserted in succession, wherein the transport device (120) comprises at least one friction clutch (110), wherein the friction clutch (110) has at least one first friction element (148) that can be coupled to a drive (186) and at least one second friction element (150) that can be coupled to the analysis tape (170), in particular a reel (118) of the analysis tape (170), wherein the first friction element (148) and the second friction element (150) are coupled by friction such that the first friction element (148) entrains the second friction element (150) in a first torque range and slips over the second friction element (150) in a second torque range, wherein the friction clutch (110) is arranged between a driven axle (122, 126) and an element surrounding the axle (122, 126), in particular a reel (118) of the analysis tape (170), **characterized in that** provision is made for at least one intermediate element between the first friction element (148) and the second friction element (150), wherein the intermediate element has elastic properties, wherein the second friction element (150) is embedded between two first friction elements (148) in the axial direction, wherein at least one axial force component acts on the second friction element (150).

2. Analytic test instrument (112) according to the preceding claim, wherein a transition between the first torque range and the second torque range is predetermined by a transition between static friction and dynamic friction between the first friction element (148) and the second friction element (150).

3. Analytic test instrument (112) according to one of the preceding claims, wherein at least one of the friction elements (148, 150) comprises at least one friction sleeve (134).

4. Analytic test instrument (112) according to one of the preceding claims, wherein the first friction element (148) and/or the second friction element (150) comprises at least one conical friction surface (164, 166).

5. Analytic test instrument (112) according to the two preceding claims, wherein the friction sleeve (134) comprises at least one friction surface (154) that is tilted with respect to the axle (122, 126), wherein the first friction element (148) comprises at least one conical friction surface (164, 166) arranged in a concentric fashion to the axle (122, 126), which conical friction surface interacts, coupled by friction, with the tilted friction surface (154).

6. Analytic test instrument (112) according to the preceding claim, wherein the conical friction surface (164, 166) comprises a first conical surface (164) arranged on a first side of the friction sleeve (134) and a second conical surface (166) arranged on an opposite side of the friction sleeve (134).

7. Analytic test instrument (112) according to one of the preceding claims, wherein the friction coupling between the first friction element (148) and the second friction element (150) is brought about via the at least one intermediate element, in particular at least one 0-ring (144, 146).

8. Analytic test instrument (112) according to one of the preceding claims, wherein provision is made for at least one adjustment element, wherein the adjustment element makes it possible to adjust a frictional force between the first friction element (148) and the second friction element (150).

9. Analytic test instrument (112) according to the preceding claim, wherein the adjustment element comprises at least one adjustment nut (138).

10. Analytic test instrument (112) according to one of the preceding claims, wherein provision is made for at least one spring element (168), wherein the spring element (168) is designed to influence a frictional force between the first friction element (148) and the second friction element (150).

11. Analytic test instrument (112) according to one of the preceding claims, wherein the same material pairs are present at each point of contact between the first friction element (148) and the second friction element (150).

12. Tape magazine (114) for use in an analytic test instrument (112) according to one of the preceding claims, wherein the tape magazine (114) comprises the at least one analysis tape (170), wherein the friction clutch (110) is wholly or partly contained within the tape magazine (114).

13. Use of a friction clutch (110) in a transport device (120) for an analysis tape (170) in an analytic test instrument (112), more particularly an analytic test instrument (112) according to one of the preceding claims relating to an analytic test instrument (112), wherein the analysis tape (170) comprises a plurality of analytic aids (172) that can be used in succession, wherein the friction clutch (110) has at least one first friction element (148) that can be coupled to a drive (186) and at least one second friction element (150) that can be coupled to the analysis tape (170), in particular a reel (118) of the analysis tape (170), wherein the first friction element (148) and the second friction element (150) are coupled by friction such that the first friction element (148) entrains the second friction element (150) in a first torque range and slips over the second friction element (150) in a second torque range, wherein the friction clutch (110) is arranged between a driven axle (122, 126) and an element surrounding the axle (122, 126), in particular a reel (118) of the analysis tape (170), **characterized in that** provision is made for at least one intermediate element between the first friction element (148) and the second friction element (150), wherein the intermediate element has elastic properties, wherein the second friction element (150) is embedded between two first friction elements (148) in the axial direction, wherein at least one axial force component acts on the second friction element (150).

## Revendications

1. Appareil de test analytique (112), présentant au moins un dispositif de transport (120) pour une bande d'analyse (170), en particulier une bande à lancettes, selon lequel la bande d'analyse (170) comprend une pluralité de moyens auxiliaires (172) analytiques qui peuvent être utilisés de manière successive, selon lequel le dispositif de transport (120) comprend au moins un accouplement à friction (110), selon lequel l'accouplement à friction (110) présente au moins un premier élément de friction (148) qui peut être accouplé avec un entraînement (186) et au moins un deuxième élément de friction (150) qui peut être accouplé avec la bande d'analyse (170), en particulier un rouleau (118) de la bande d'analyse (170), selon lequel le premier élément de friction (148) et le deuxième élément de friction (150) sont reliés par une force de friction, de telle sorte que le premier élément de friction (148) entraîne avec lui le deuxième élément de friction (150), dans une première plage de vitesse de rotation, et glisse sur le deuxième élément de friction (150), dans une deuxième plage de vitesse de rotation, selon lequel l'accouplement à friction (110) est disposé entre un axe (122, 126) entraîné et un élément qui entoure l'axe (122, 126), en particulier un rouleau (118) de la bande d'analyse (170), **caractérisé en ce qu'**au moins un élément intermédiaire est prévu entre le premier élément de friction (148) et le deuxième élément de friction (150) selon lequel l'élément intermédiaire présente des propriétés élastiques, selon lequel le deuxième élément de friction (150) est niché entre deux premiers éléments de friction (148) dans la direction axiale, selon lequel au moins une composante axiale de force agit sur le deuxième élément de friction (150).

2. Appareil de test analytique (112) selon la revendication précédente, selon lequel il existe un passage entre la première plage de vitesse de rotation et la deuxième plage de vitesse de rotation par l'intermédiaire d'un passage entre une friction par adhérence et une friction par glissement entre le premier élément de friction (148) et le deuxième élément de friction (150).

3. Appareil de test analytique (112) selon l'une des revendications précédentes, selon lequel au moins l'un des éléments de friction (148, 150) comprend au moins un manchon à friction (134).

4. Appareil de test analytique (112) selon l'une des revendications précédentes, selon lequel le premier élément de friction (148) et/ou le deuxième élément de friction (150) comprennent au moins une surface de friction (164, 166) de forme conique.

5. Appareil de test analytique (112) selon les deux revendications précédentes, selon lequel le manchon à friction (134) comprend au moins une surface de friction (154) qui est basculée par rapport à l'axe (122, 126), selon lequel le premier élément de friction (148) comprend au moins une surface de friction (164, 166) qui est de forme conique et qui est disposée de manière concentrique par rapport à l'axe (122, 126), laquelle au moins une surface de friction interagit avec la surface de friction (154) qui est basculée, par l'intermédiaire d'une force de friction.

6. Appareil de test analytique (112) selon la revendication précédente, selon lequel la surface de friction (164, 166) de forme conique comprend une première surface (164) de forme conique, laquelle est disposée sur une première face du manchon à friction (134), et comprend une deuxième surface (166) de forme conique, laquelle est disposée sur une face opposée du manchon à friction (134).

7. Appareil de test analytique (112) selon l'une des revendications précédentes, selon lequel la liaison par friction est réalisée entre le premier élément de friction (148) et le deuxième élément de friction (150), par l'intermédiaire de l'au moins un élément intermédiaire, en particulier par l'intermédiaire d'au moins une bague torique (144, 146).

8. Appareil de test analytique (112) selon l'une des revendications précédentes, selon lequel au moins un élément de réglage est prévu, selon lequel une force de friction peut être réglée entre le premier élément de friction (148) et le deuxième élément de friction (150) au moyen de l'élément de réglage.

9. Appareil de test analytique (112) selon la revendication précédente, selon lequel l'élément de réglage comprend au moins un écrou de réglage (138).

10. Appareil de test analytique (112) selon l'une des revendications précédentes, selon lequel au moins un élément à ressort (168) est prévu, selon lequel ledit élément à ressort (168) est mis en place en vue d'exercer une influence sur une force de friction située entre le premier élément de friction (148) et le deuxième élément de friction (150).

11. Appareil de test analytique (112) selon l'une des revendications précédentes, selon lequel il existe des appariements de matériaux identiques au niveau de chaque point de contact se trouvant entre le premier élément de friction (148) et le deuxième élément de friction (150).

12. Magasin de bandes (114) destiné à une utilisation dans un appareil de test analytique (112) selon l'une des revendications précédentes, selon lequel le magasin de bandes (114) comprend l'au moins une bande d'analyse (170), selon lequel l'accouplement à friction (110) est contenu, en totalité ou en partie, dans le magasin de bandes (114).

13. Utilisation d'un accouplement à friction (110) dans un dispositif de transport (120) pour une bande d'analyse (170) dans un appareil de test analytique (112), en particulier un appareil de test analytique (112) selon l'une des revendications précédentes qui concernent un appareil de test analytique (112), selon lequel la bande d'analyse (170) comprend une pluralité de moyens auxiliaires (172) analytiques qui peuvent être utilisés de manière successive, selon lequel l'accouplement à friction (110) présente au moins un premier élément de friction (148), qui peut être accouplé avec un entraînement (186), et comprend au moins un deuxième élément de friction (150) qui peut être accouplé avec la bande d'analyse (170), en particulier un rouleau (118) de la bande d'analyse (170), selon lequel le premier élément de friction (148) et le deuxième élément de friction (150) sont reliés par une force de friction, de telle sorte que le premier élément de friction (148) entraîne avec lui le deuxième élément de friction (150), dans une première plage de vitesse de rotation, et glisse sur le deuxième élément de friction (150), dans une deuxième plage de vitesse de rotation, selon lequel l'accouplement à friction (110) est disposé entre un axe (122, 126) entraîné et un élément qui entoure l'axe (122, 126), en particulier un rouleau (118) de la bande d'analyse (170), **caractérisé en ce qu'**au moins un élément intermédiaire est prévu entre le premier élément de friction (148) et le deuxième élément de friction (150), selon lequel ledit élément intermédiaire présente des propriétés élastiques, selon lequel le deuxième élément de friction (150) est niché entre deux premiers éléments de friction (148) dans la direction axiale, selon lequel au moins une composante axiale de force agit sur le deuxième élément de friction (150).
